# EUROPEAN PATENT SPECIFICATION

(11) **EP 3 906 040 B1**
(45) Date of publication and mention of the grant of the patent: **08.04.2026**
(21) Application number: 19832915.3
(22) Date of filing: 19.12.2019
(51) Int. Cl.: A61K 36/71, A61K 36/28, A61P 35/00

(54) **COMBINATION OF CIMICIFUGA AND PETASITES EXTRACTS FOR ANTICANCER THERAPY AND PROPHYLAXIS**
KOMBINATION AUS UCIMICIFUGA- UND PETASIT-EXTRAKTEN FÜR ANTIKREBSTHERAPIE UND -PROPHYLAXE
COMBINAISON D'EXTRAITS DE CIMICIFUGA ET DE PETASITES POUR LE TRAITEMENT THÉRAPEUTIQUE OU PROPHYLACTIQUE DU CANCER

(30) Priority: 31.12.2018 EP 18215929
(43) Date of publication of application: 10.11.2021
(73) Proprietor: Max Zeller Söhne AG, 8590 Romanshorn (CH)
(72) Inventor: DREWE, Jürgen, 8082 Kreuzlingen (CH); HASLER, Samuel, 8500 Frauenfeld (CH); BOONEN, Georg, 8590 Romanshorn (CH)
(74) Representative: Kasche & Partner
(86) International application number: PCT/EP2019/086205
(87) International publication number: WO 2020/141083

(56) References cited:
- WO-A1-2015/018471
- CN-B- 101 028 322
- DE-C1- 19 652 183

## Description

The present invention is directed to the novel use of an extract combination comprising extracts from a *Cimicifuga species,* e.g. *Cimicifuga racemosa* (also termed *Actaea racemosa L;* black cohosh) and a *Petasites* species, e.g. *Petasites hybridus,* for the therapeutic or prophylactic treatment of cancer. Also, the invention pertains to the use of such extract combinations for preparing a medicament, corresponding pharmaceutical compositions, comprising a combination of an extract from *Cimicifuga sp*. and an extract from *Petasites sp.,* as well as a method for the therapeutic or prophylactic treatment of cancer, preferably by oral administration of the extract combination or pharmaceutical composition.

*Cimicifuga racemosa* extract is commonly used in the treatment of menopausal symptoms such as hot flashes and sweating due to hormonal changes, such as estrogen depletion. The extract has the benefits of hormonal replacement therapy without the increased risk for breast cancer or thromboembolic events. In fact, *Cimicifuga racemosa* extract is known for its positive benefit-risk profile and is listed in a monograph by the Herbal Medicinal Product Committee (HMPC) of the European Medicines Agency (EMA).

WO 99/47149 describes the use of *Cimicifuga* extracts for use as medicaments for selective treatment of cardiovascular diseases and climacteric discomfort. Seidlova-Wuttke et al. (Phytomedicine, 19, 2012, 846-853) discloses the use of *Cimicifuga racemosa* extracts for reducing abdominal fat depots (hyperlipidemia) and thus preventing metabolic syndrome (MB) in ovariectomized (ovx) rats, mimicking a postmenopausal situation.

WO 2005/002609 A1 teaches methods for the preparation of plant extracts, in particular from *Cimicifuga racemosa* using extraction agents together with solution mediators such as organic polymers, in particular poly(vinylpyrrolidone).

WO 98/26791 A1 teaches the use of an extract of *Cimicifuga racemosa* for preparing a medicament for treating estrogen-dependent tumors in combination with a medicament containing an anti-estrogenic active agent such as tamoxifen or genistein.

Borrelli et al. (Eur. J. Clin. Pharmacol., 58, 2002, 235 - 241) provides a systematic review of the clinical efficacy of *Cimicifuga racemosa* in many medical indications.

The *Cimicifuga racemosa* extract for Cimifemin^{®} uno (manufactured by Max Zeller Soehne AG, Romanshorn, Switzerland, also called Ze 450) is an extract produced by a procedure comprising root extraction by an aqueous-ethanolic solvent (60 % V/V) containing in minimum 6 % (m/m) triterpene glycosides, expressed as 27-deoxyactein and calculated with reference to the native dried extract. The extract features over 20 triterpensaponines with actein and 27-deoxyactein being most prominent. It is approved for the treatment of peri-menopausal complaints in doses of 6.5 to 13 mg per day (1 tablet per day) in many European countries.

The extract was also shown to increase AMPK phosphorylation *in vitro* and ameliorated metabolic disorders in *ob*/*ob* mice and in ovariectomized rats *in vivo,* thereby indicating beneficial metabolic effects. EP2874637 A1 discloses an extract from *Cimicifuga racemosa* for use in the therapeutic or prophylactic treatment of diabetes, in particular diabetes mellitus type 2.

C. Speirs et al. (2018, Pharmacol Res, 128, 88-100) provide a review on the potential use of AMPK activators to treat several haematological malignancies containing mutationally activated JAK, such as myeloproliferative neoplasms or acute lymphoblastic leukemia.

The plant *Petasites (Petasites hybridus (L.) Gaertn., B. Mey. et Scherb.)* is commonly also referred to as Butterbur and *Petasites* extracts can be obtained from the plant, in particular from the leaves and/or the rhizome. The production of *Petasites* extracts is well known in the art. However, depending on the extraction protocol the extracts can comprise pyrrolizdine-alkaloids which can be hepatotoxic, mutagenic and carcinogenic.

*Petasites* extracts are known to display spasmolytic and analgesic properties. DE-A1 198 38 848 discloses *Petasites* extracts for use in the treatment of gastrointestinal diseases, asthma, pollinosis, dysmenorrhoea, eczemas, migraine, psoriasis, high blood pressure and/or spasms. EP 1 499 334 B1 describes the use of a polar *Petasites* extract for use in the treatment of pains. S. A. Steiert et. al. (2017, Biofactors, 43, 388-399) describes the anti-inflammatory effects of the petasin phytodrug Ze 339 involving inhibition of the STAT signalling pathway. The use of *Petasites* extracts for treating allergic rhinitis has been clinically verified and is described in the art. For further information on the medical utility of *Petasites* extracts reference is made to, e.g. Schapowal et al., 2002, BMJ, 324, 144-6; Thomet et al., 2002, Intern. Immunopharmacol.; Brattström et al., 2003, Phytomedicine, 10, Suppl 4, 50-2; Schapowal et al., 2004, Arch. Otolaryngol. Head Neck Surg., 130, 1381-6; Schapowal et al., 2005, Phytother. Res., 19, 530-7; Keusch et al., 2004, Ars Medici; Käufeler et al., 2006, Adv. Ther., 23, 373-84; Brattström et al., 2010, Phytother. Res., 24, 680-5; Dumitru et al., 2011, J. Allergy Clin. Immunol., 127, 1515-21; and Nebel et al., 2014, Planta Med., 80).

The active ingredients in *Petasites* include mainly sesquiterpenes, in particular petasin, isopetasin and neopetasin that feature high lipophilicity and good bioavailability.

The *P. hybridus* leaf native extract in Tesalin^{®} of Max Zeller Soehne AG (Romanshorn, Switzerland), also called Ze 339, is a "new herbal entity" since extracts from *Petasites* leaves have not yet been used medically. The extract is prepared by means of subcritical carbon dioxide extraction from the leaves of a chemovariant of *Petasites hybridus (Petasites hybridus (L.) Gaertn., B. Mey. et Scherb.).* It is standardized for petasins (sum of petasin, isopetasin and neopetasin) and each Tesalin^{®} tablet contains 8 mg petasins. The daily dosage is 2 to 3 tablets. In 2017 Tesalin^{®} was switched from list B (Rx product) to list C (OTC) in the Swiss list of approved medicines. Tesalin^{®} / Ze 339 is one option among suitable Petasites alternatives and is the most preferred extract and pharmaceutical composition for practicing the present invention.

Metformin, a methylated biguanide, is mostly known for its anti-diabetic effect for the treatment of type 2 diabetes based on the reduction of blood sugar levels. In recent years metformin has been investigated for possible cancer prevention and therapy due to the observation of a decreased risk of the occurrence of various types of cancers, especially of pancreas, colon and hepatocellular cancer, which led to the further investigation of several mechanisms underlying the proposed anti-cancer effect of metformin as well as its activity against various types of cancer both in diabetic and non-diabetic populations (Kasznicki et al, Ann. Transl. Med. 2014 June; 2(6): 57). Metformin leads to indirect AMPK-activation, which is related to the direct inhibition of complex I of the mitochondrial respiratory chain (Viollet et al., Clin. Sci. (Lond.) 2012, 122 (6): 253-270T; Kawashima & Kirito, Experimental Hematology 2016, 44(12):1156-1165). The antiproliferative effect of Metformin on cancer cells was partly explained through an increased energetic vulnerability of cancer cells which have been switched to aerobic glycolysis, the so-called "Warburg" effect (Andrzejewski et al., Cancer & Metabolism 2014, 2:12; Wheaton et al., eLife, 20143:e02242). Metformin further inhibits the mammalian target of rapamycin (mTOR) that inhibits cancer cell proliferation (Ben Sahra et al., Cancer research 2011, 71(13): 4366-4372; Jara & Lopez-Munoz, Pharmacological Research: the official journal of the Italian pharmacological society 2015, Ikhlas & Ahmad, Life Sci., 2017, 185:53-62). Chae et al. (Oncotarget, 2016 7(26)) report current clinical studies for investigating a possible use of metformin for cancer treatment.

The objective technical problem underlying the present invention is the provision new and improved medical means for the prophylaxis and/or treatment of cancer.

It was surprisingly found that an extract combination comprising extracts from *Cimicifuga sp.* and *Petasites sp.* is particularly suitable for use in the therapeutic or prophylactic treatment of cancer. The synergistic effects of both extracts in combination was demonstrated experimentally further below in a human erythroleukemia cell line carrying the JAK2V617F mutation.

Wagner H. (2011, Fitoterapia 82, 34-37) acknowledges the isobologram as a practicable and effective method for demonstrating drug synergism. In the experimental section below a synergism for the combination of an extract from *Petasites sp.* and an extract of *Cimicifuga sp.* is proven using such isobologram.

In a **first aspect** the present invention is directed to an extract combination comprising extracts from *Cimicifuga sp.* and *Petasites sp.* for use in the therapeutic or prophylactic treatment of cancer.

In the extract combination for practicing the invention the *Cimicifuga* extract may be obtained from *Cimicifuga racemosa.* Further exemplary species of *Cimicifuga* (*syn. Actea*) for use in the present invention are C. *racemosa* L., *C*. *americana* Michx., *C*. *foetida* L *(syn. A. cimicifuga), C. dahurica* (Turcz) Maxim, C. *heracleifolia* Kom., *Actea cordifolia, A. pachypoda, A. podocarpa or A. rubra.*

For the inventive extract combination *Petasites* extract may be obtained from *Petasites hybrid-dus.* Further exemplary species of *Petasites* plant for use in the present invention are *Petasites albus, Petasites amplus, Petasites fragrans Petasites formosanus, Petasites frigidus, Petasites georgicus, Petasites japonicus, Petasites laevigatus, Petasites kalbikianus, Petasites niveus, Petasites paradoxus, Petasites pyrenaicus, Petasites tricholobus, Petasites radiates, Petasites sagittatus* and *Petasites spurius,* preferably *Petasites hybridus.*

The extract combination for practicing the present invention is generally suitable for treating cancer, i.e. for preventing and/or inhibiting cancer growth and/or spreading. For example, the extract combination is suitable for therapeutically and/or prophylactically treating cancers selected from the group consisting of malignant neoplasms of the lip, oral cavity, pharynx, digestive organs, respiratory system, intrathoracic organs, bone, articular cartilage, skin, connective tissue, soft tissue, breast, female genital organs, male genital organs, urinary organs, eye, brain and central nervous system, endocrine glands, lymphoid tissue, haematopoietic tissue, as well as benign neoplasms.

The extract combination for practicing the invention is particularly suitable for therapeutically and/or prophylactically treating cancer, optionally selected from the group of cancers consisting of polycythaemia vera (PV), myelodysplastic syndromes (MDS) and other neoplasms of lymphoid and haematopoietic tissue, such as essential thrombocythaemia (ET), primary myelofibrosis (PMF), chronic myelogenous leukemia (CML), chronic neutrophilic leukemia (CNL), juvenile myelomonocytic leukemia (JML), chronic eosinophilic leukemia (CEL), non-Hodgkin's lymphoma or multiple myeloma, as well as breast cancer, prostate cancer, pancreas cancer, hepatocellular cancer, colon cancer, lung cancer and renal cancer.

Generally, the term *"extract"* as used in the art and herein is intended to refer to any form of the product of extraction minus the extracting agent, e.g. minus a major part, most or all of the extracting agent, and, thus, refers to any product of extraction regardless of the final chemical composition or physical form, e.g. liquid, viscous, pasty or solid. Of course, a plant extract for pharmaceutical use is understood to relate to a plant extract product comprising the active plant agent(s) in a physiologically effective form and amount. Typically, a solvent-based extraction, e.g. aqueous, non-aqueous, liquid, gaseous, critical gas, e.g. sub- or supercritical carbon dioxide extraction, will result in extracted components that are optionally separated from the remaining raw materials, e.g. separated from the remaining solid plant materials, and which components can optionally be further processed, e.g. (partially) purified.

The extract of *Cimicifuga sp.* for practicing the present invention can be prepared by a number of well-known conventional and non-conventional methods. Preferably it is prepared by a method comprising the steps:
(i) providing plant material from *Cimicifuga sp.,* preferably *Cimicifuga racemosa,*
(ii) treating the plant material with an extraction agent, optionally an aqueous-ethanolic solvent; and
(iii) concentrating the extract.

It is proposed as option that the extract resulting from above step (ii) is concentrated in the presence of a solution mediator, preferably an organic polymer capable of being colloidally dispersible or soluble in the extraction agent, more preferably an organic polymer having an average molecular mass of at least about 1000 Dalton units, for example polyvinylpyrrolidone (PVP).

In one specific embodiment of this aspect, the *Cimicifuga* extract for practicing the invention is obtained from *Cimicifuga racemosa* L., *C*. *americana Michx., C. foetida* L. (syn. *A. cimicifuga*)*, C. dahurica* (Turcz) Maxim, *C. heracleifolia* Kom., *A. cordifolia, A. pachypoda, A. podocarpa* and *A. rubra, preferably C. racemosa,* and/or the extract is suitable and optionally intended for oral administration.

*Petasites* extracts for practicing the present invention can be obtained conventionally by methods generally known to the experts in the field of plant extraction. Depending on the selected type and mode of extraction, a *Petasites* extract can also comprise to a varying extent toxic pyrrolizidine-alkaloids and pyrrolizidine-alkaloid derivatives such as N-oxides. In a preferred embodiment, the *Petasites* extract in the extract combination for practicing the present invention is substantially free of pyrrolizidine-alkaloids including any derivatives of pyrrolizidine-alkaloids such as, e.g. N-oxides. Substantially free of pyrrolizidine-alkaloids means that the *Petasites* extract comprises non-toxic amounts of pyrrolizidine-alkaloids, preferably ≤ 8 ppb pyrrolizidine-alkaloids, more preferably ≤ 4 ppb pyrrolizidine-alkaloids, most preferably < 2ppb pyrrolizidine-alkaloids.

Generally, *Petasites* extracts can be distinguished into polar and non-polar *Petasites* extracts. For example, the extract for practicing the invention can be a non-polar *Petasites* extract, optionally one produced by extraction with liquid carbon dioxide, preferably by extraction with liquid carbon dioxide at subcritical temperature conditions (i.e. subcritical carbon dioxide). For carbon dioxide the critical point is 31.1 °C and 73.8 bar. Subcritical temperature conditions in the context of carbon dioxide extraction are preferably extraction temperatures ≤ 31 °C at 73.8 bar, more preferably extraction temperatures between 0 to 30 °C (the pressure depends on the temperature) to reach the liquid state. For example, an extraction of *Petasites* using a subcritical carbon dioxide extraction protocol to obtain non-polar *Petasites* extracts for practicing the present invention is described in European patent EP 1 023 079 B1.

In a preferred embodiment the extract of *Petasites sp.* for use in the present invention is one, wherein the extract is prepared by a method comprising the steps:
(i) providing plant material from *Petasites sp.,* preferably *Petasites hybridus,*
(ii) treating the plant material with subcritical carbon dioxide, and
(iii) concentrating the extract.

In an alternative embodiment, the extract for use in the invention is a polar *Petasites* extract, e.g. a polar *Petasites* extract which is substantially free of pyrrolizidine-alkaloids, optionally a polar *Petasites* extract having an Rf value (TLC, silica gel 60, eluent toluene/ethyl acetate 93:7) of 0 to 0.21, e.g. as described in European patent EP 1 499 334 B1. The principle method of TLC analysis is well known to the skilled person and is, for example, described in Hans Rudolf Christen and Fritz Vögtle, "Organische Chemie - von den Grundlagen zur Forschung - Volume 1, 36-37, 1988.

In a preferred embodiment, the *Petasites* extract for use in combination with a *Cimicifuga* extract is polar or non-polar, and/or substantially free of pyrrolizidine-alkaloids.

The *Petasites* extract for combination with a *Cimicifuga* extract is not limited to any specific *Petasites* species origin because substantially all of the *Petasites* plants comprise qualitatively the same physiologically active ingredients. Therefore, and in a further specific embodiment, the invention is directed to the use of *Petasites* extracts in combination with a *Cimicifuga* extract, wherein the *Petasites* extract is obtained from plants and/or parts of plants selected from the group consisting of *Petasites hybridus, Petasites albus, Petasites amplus, Petasites fragrans, Petasites formosanus, Petasites frigidus, Petasites georgicus, Petasites japonicus, Petasites laevigatus, Petasites kalbikianus, Petasites niveus, Petasites paradoxus, Petasites pyrenaicus, Petasites tricholobus, Petasites radiates, Petasites sagittatus* and *Petasites spurius.*

In a preferred embodiment of this aspect the extract combination for practicing the invention is made from *Petasites hybridus* and *Cimicifuga racemosa* and optionally the extract combination is suitable for oral administration.

In a **second aspect,** the present invention relates to the use of a combination of extracts from *Cimicifuga sp.* and *Petasites sp.,* optionally prepared by separate or combined extraction, for preparing a medicament for the therapeutic or prophylactic treatment of cancer.

For this use *Cimicifuga sp.* may be *Cimicifuga racemosa* and/or the *Petasites sp.* may be *Petasites hybridus.*

Specific but non-limiting *Cimicifuga* plant species as well as specific but non-limiting *Petasites* plant species for preparing the extract combination for practicing the inventive use are listed above and in the claims. The same, specific but non-limiting cancers for this use are listed above and in the claims.

Preferably, the extract combination medicament for use in the present invention is used in combination with further anti-cancer agents. For example, the extract combination can be used for adjuvant treatment of any type of cancer. In a particular embodiment this aspect relates to a use, wherein the extract combination is made from *Cimicifuga racemosa* and *Petaistes hybridus* and the extract combination is for oral administration.

**In a third aspect,** the present invention pertains to a pharmaceutical composition comprising a combination of an extract from *Cimicifuga sp.* and an extract from *Petasites sp.,* and optionally at least one pharmaceutically acceptable excipient for the therapeutic or prophylactic treatment of cancer. The cancers for treatment with this composition may be selected from the group consisting of malignant neoplasms of the lip, oral cavity, pharynx, digestive organs, respiratory system, intrathoracic organs, bone, articular cartilage, skin, connective tissue, soft tissue, breast, female genital organs, male genital organs, urinary organs, eye, brain and central nervous system, endocrine glands, lymphoid tissue, haematopoietic tissue, as well as benign neoplasms and neoplasms of uncertain or unknown behaviour. The composition is particularly suitable for treating a cancer selected from but not limited to the group consisting of polycythaemia vera (PV), myelodysplastic syndromes (MDS) and other neoplasms of lymphoid and haematopoietic tissue, such as essential thrombocythaemia (ET), primary myelofibrosis (PMF), chronic myelogenous leukemia (CML), chronic neutrophilic leukemia (CNL), juvenile myelomonocytic leukemia (JML), chronic eosinophilic leukemia (CEL), non-Hodgkin's lymphoma or multiple myeloma, as well as breast cancer, prostate cancer, pancreas cancer, hepatocellular cancer, colon cancer, lung cancer and renal cancer.

Specific *Cimicifuga* and *Petasites* extracts for practicing the claimed pharmaceutical compositions are listed above.

For example, the pharmaceutical composition for use in the invention can also be employed for adjuvant treatment, i.e. in combination with at least one further anti-cancer agent. In a further embodiment the pharmaceutical composition of the invention is one wherein the *Cimicifuga sp.* is *Cimicifuga racemosa,* and/or *Petasites sp.* is *Petasites hybridus,* and/or the pharmaceutical composition is for oral administration.

An extract combination or a pharmaceutical composition, i.e. a medicament for use according to the invention, comprises an extract of *Cimicifuga* and an extract of *Petasites* in a pharmaceutically effective amount suitable for administration, optionally formulated together with conventional pharmaceutically acceptable additives, carriers, adjuvants and excipients and/or further pharmaceutically active agents. Such carriers, adjuvants and excipients include, for example, ion exchangers, alumina, aluminum stearate, lecithin, serum proteins, buffer substances, water, salts, electrolytes and cellulose-based substances. Also, maltodextrin or silica can be used to produce a free-flowing dry extract which is particularly suitable for further processing. Controlled release dosage forms with or without immediate release portions are also envisaged. Methods for preparing such dosage forms are known (see, for example, H. C. Ansel and N. G. Popovish, Pharmaceutical Dosage Forms and Drug Delivery Systems, 5th ed., Lea and Febiger (1990)). Dosage levels and requirements are well-recognized in the art and may be selected by those of ordinary skill in the art from available methods and techniques suitable for a particular patient. One skilled in the field of preparing formulations can readily select the proper form and mode of administration for a *Cimicifuga* extract and *Petasites* extract for use in the present invention depending upon the particular characteristics of the extract product selected, the cancer disease or cancer-related condition to be treated, the stage of the disease or condition, the specific patient and other relevant circumstances (see. e.g. Remington's Pharmaceutical Sciences, Mack Publishing Co. (1990)).

As the person skilled in the art will appreciate that lower or higher doses may be required depending on particular factors. For instance, specific doses and treatment regimens will depend on factors such as the patient's general health profile, the severity and course of the patient's disorder or disposition thereto, and the judgment of the treating physician which includes interaction potentials with other needed medication.

Next to pharmaceutically acceptable agents, the extract combination or pharmaceutical composition for use according to the invention can also comprise additional physiologically active agents. Hence and in a further preferred embodiment, the pharmaceutical composition for use in the invention may additionally comprise at least one ingredient selected from the group consisting of antiphlogistics, analgesic and fever reducing agents such as salicylates, antidiabetics such as metformin, inhibitors of glycolysis such as 2-deoxyglucose or syrosingopine, kinase inhibitors, for example but not limited to Ruxolitinib, Nilotinib, Tandutinib, Imatinib, Sorafenib, Dasatinib, Ibrutinib, Bosutinib, Ponatinib, Dovitinib, Lestaurtinib, Midostaurin or Radotinib, interferons, hydroxyurea, extracts, preferably from *Chamomilla recutita, Curcuma longa, Curcuma canthorrhiza, pomegranate, Salix alba, Salix purpurea, Salix daphenoides, Tanacetum parthenium, Moringa peregrina, Saffron crocus, Zingiber officinale, Rosmarinus officinalis, Camellia sinensis, Coffea Arabica, Coffea robusta, Vitis vinifera,* trace elements, preferably salts of magnesium or calcium, vitamins, preferably vitamin A, C, D or E as well as antioxidants, preferably lutein, lycopene, zeaxanthin or bioflavonoids. The extract combination for use in the present invention can be administered the same way as other chemical drugs or plant extracts and pharmaceutical compositions thereof.

For example, the extract combination or pharmaceutical composition for use in the present invention can be administered orally, topically, subcutaneously, intranasally, by inhalation or as a spray, preferably orally, by inhalation, topically or intranasally, rectally or vaginally.

It is preferred that the extract combination or pharmaceutical composition for practicing the invention is in a dosage form selected from the group consisting of a spray, an aerosol, a foam, an inhalant, a powder, a tablet, a capsule, a soft capsule, a tea, a syrup, a granule, a chewable tablet, a salve, a cream, a gel, a suppository, a lozenge, a liposome composition or a solution, preferably a time-release or sustained-release dosage form of the above.

While all types of administration of the inventive medicament are believed to be suitable for use in the invention, oral, topical, inhalatory and intranasal administrations are preferred for many purposes, and the extract combination can be used as such or in dilution with solid and/or liquid additives and/or contain additives, carriers, adjuvants, excipients and/or other pharmacologically active constituents.
In a further specific and non-limiting embodiment, the extract combination or pharmaceutical composition for use in the invention is a dosage form selected from the group consisting of a tablet, a capsule, a powder, a granule, a tea, a syrup, an aerosol, an inhalant, a spray, and a time-release or sustained-release dosage form of the above.

In a **fourth aspect** the present invention relates to a method for the therapeutic or prophylactic treatment of cancer, comprising the step of administering
(i) a combination of an extract from *Cimicifuga sp.* and an extract from *Petasites sp.,* or
(ii) a pharmaceutical composition comprising said extracts,
in a physiologically effective amount to a patient in need thereof.

Specific but non-limiting cancer types for this aspect are taught above and in the claims. In a further embodiment, the extract combination or composition is administered intravenously, intraperitoneally or orally, preferably orally. The extract combination or pharmaceutical composition can be administered alone or in combination with one or more further active agents, preferably at least one further anti-cancer agent. In a further embodiment the composition is administered as an adjuvant in combination with at least one further anti-cancer agent.

In a particular embodiment relating to all aspects of the invention the extract combination for practicing the invention may be obtained from plant material from *Cimicifuga racemosa* and plant material from *Petasites hybridus.*

In the following the invention will be detailed with reference to this species, however, the invention is not limited to any particular *Cimicifuga* or *Petasites* species.

While extracts of *Cimicifuga racemosa* obtained from various parts of that plant, e.g. from leaves, roots, stems, branches, or seeds, and/or by different extraction methods and extraction solvents are suitable for the invention, a preferred extract is one obtained from root material. Another preferred extract is one prepared according to the method disclosed in EP 1 644 015 mentioned above. Extracts of *Cimicifuga racemosa* prepared by other methods are suitable for the invention, too.

In general, methods of producing *Cimicifuga* extracts are based upon treatment of plant material with an aqueous alkanolic extracting agent in order to obtain a raw or primary extract which, after an optional treatment for the removal of fines, e.g. by sedimentation or filtration, so as to obtain a primary extract which contains the extracting agent and the plant constituents that are soluble in the extracting agent.

Typically, the primary extract is then concentrated by partial evaporation of the extracting agent in order to remove its more volatile components, e.g. an alkanolic constituent containing 1 - 4 carbon atoms in the alkanol group, plus some water, and to form what is called a concentrated extract, typically containing 5 - 50 % by volume, of residual solvent, i.e. water. Upon further removal of solvent, a solid, pasty or liquid material is obtained that is substantially free of the solvent used for extraction of the plant material. This product can then be used as such or be processed to produce specific application forms, e.g. pills, lotions, solutions, powders etc., optionally adding whatever adjuvants, additives, coating components and the like are needed for the final medicinal product.

Generally, extracts from *Cimicifuga racemosa* suitable for the purposes of the present invention will contain actein, 27-deoxyactein and cimiracemoside C. Actein is a xyloside of acetyl acteol with a 16,23:23,26:24,25-triepoxy side chain. 27-Deoxyactein is used as a standard for quantitative determination of the compounds mentioned above. A further known group of active ingredients are aromatic acids such as ferulic acid, hesperatinic acid and acyl caffeic acid. Further, the following esters of hydroxy cinnamic acid have been isolated from aqueous ethanolic extracts of *Cimicifuga racemosa:* fucinolic acid and the cimicifugaic acids A, B, E and F. Flavonoides, biochanine A, formononetine and camphorol have also been reported. Other ingredients are tannins, resins and fatty acids (oleic acid, linolic acid, linolenic acid and palmitinic acid). It is noted that the content of ferulic acid in *Cimicifuga racemosa* is rather low.

With such a large number of constituents extraction conditions tend to have a substantial impact on the composition of the resulting extracts and any unintended change of the proportional makeup is undesirable.

Various additives, soluble or insoluble in the extraction agent, have been suggested and used to improve the concentration step in the preparation of the extract. While various additives such as organic acids or gelatine may be used to prevent unintended precipitation when concentrating the extract, the preferred additive, also termed "solution mediator" for use according to the present invention is polyvinylpyrrolidone (PVP) as specified in the above-mentioned EP 1 644 015.

The extract obtained by evaporation of the extracting solvent can be subjected to a further treatment for substantially removing any residual extraction agent, preferably by spray drying to obtain an essentially dry product.

The addition of PVP in a preferred embodiment of the present invention does not detract from using the extract in a medical treatment or as constituent of a medicament because PVP is admitted under current food and drug regulations for pharmaceutical use in most countries.

An effective amount of PVP as solution mediator generally is in the range of about 5 % by weight to about 50 % by weight based on the weight of the final extract. A range of about 15 % by weight to about 35 % by weight is preferred for many purposes.

The extraction agent is preferably a polar substance such as water and/or a normally liquid alkanol, e.g. methanol, ethanol, propanol, butanol. Mixtures of such C_{1 - 4} alcohols with each other and/or with water are even more preferred. Mixtures of from about 40 to about 80 % by weight of such an alkanol, preferably ethanol with about 60 to about 20 %, by weight of water are particularly preferred.

The extraction procedure may be carried out in a conventional apparatus. In any case, the solution mediator is added prior to any significant removal of extraction agent. Such removal or concentration can be effected in a known manner, e.g. by distillation at an elevated temperature and/or reduced pressure taking care to avoid conditions that could affect the components of the extract. Concentration may be effected at constant or varying conditions of temperature and/or pressure.

A preferred way of producing the extract is carried out by extraction of the dry plant material with an ethanol-water mixture containing about 50 - 60 % by volume of ethanol und about 50 - 40 % by volume of water. Upon concentration the alkanolic constituents plus any water of an azeotropic mixture is removed until a solids concentration of about 25 - 50 % by weight is reached. The concentrated extract obtained may then be processed in any conventional manner, e.g. by spray-drying to form a dry product. During such a final concentration step, e.g. upon spray-drying the solution mediator PVP additionally acts as a spray-drying adjuvant. Generally, the PVP has the effect that a homogeneous consistence of the extract is maintained in all stages of the concentration process.

An extract combination, pharmaceutical composition, i.e. medicament for use according to the invention normally contains the extract of *Cimicifuga sp.,* preferably *Cimicifuga racemosa,* in an amount suitable for administration with or without conventional and well-known components of medicaments which, in turn, may but need not be pharmaceutically active. On the other hand, a pharmaceutical composition according to the invention may substantially consist of the novel extract combination, e.g. in a liquid or solid form and with or without a diluent.

While all types of application of the combination medicament are believed to be suitable for practicing the invention, oral administration is preferred for many purposes, and the extract combination can be used as such or in dilution with solid and/or liquid additives and/or contain additives or adjuvants and/or other pharmacologically active constituents. Preparation of pharmacologically suitable compositions is standard and does not require detailed explanation.

A preferred dosage for the extract of *Cimicifuga* is in the range of about 1 to about 500 mg of extract per day, preferably about 1 to about 200 mg/day, more preferred about 1 to about 100 mg /day, most preferred about 2 to about 50 mg/day, depending of course and the intensity of treatment required. For example, Cimifemin^{®} uno and Cimicemin^{®} forte contain 6.5 and 13 mg *Cimicifuga racemosa* extract Ze 450, respectively and are administered orally once a day. The extract of *Petasites* in the combination may be 5 to 600 mg act, preferably 10 to 500 mg, more preferably 20 to 100 mg, depending of course and the intensity of treatment.

The term "treatment" as used herein relates to the prophylactic and/or therapeutic treatment of a disease or medical condition. For example, the composition for use according to the present invention can be administered before or after cancer diagnosis in order to prevent, inhibit and/or alleviate cancer-induced physiological consequences. For example, the composition for use in the present invention can be used to treat benign cells likely to turn into malignant cells.

The term "polvinylpyrrolidone" is intended to include any PVP product admitted as a food or drug additive.

Numeric indications used herein and preceded by a qualifier such as "about" are intended to include a margin of error of ± 50%, preferably ± 20%.

In the following the invention will be illustrated in more detail by practical examples and with reference to figures, none of which are to be interpreted as limiting the scope of the invention beyond the claims as appended.

### Figures

**Fig. 1** is a chart of the results of example 1.1 demonstrating the anti-cancer effect of 3 concentrations of *Cimicifuga* extract Ze 450 on the growth of human PC-3 prostate adenocarcinoma cells over 16 days.
**Fig. 2** is a chart of the results of example 1.2 demonstrating the anti-cancer effect of 3 concentrations of *Cimicifuga* extract Ze 450 on the growth of human human LS-180 colorectal adenocarcinoma cells over 16 days.
**Fig. 3** is a chart of the results of example 1.3 demonstrating the anti-cancer effect of *Cimicifuga* extract Ze 450 on the growth of human HEL erythroleukemia cells during a time period of 10 days in comparison to positive (Metformin, Ruxolitinib and Hydroxyurea) and negative (untreated and vehicle) controls.
**Fig. 4** is a chart of the results of example 2.1 demonstrating the anti-cancer effect of 3 concentrations of *Petasites* extract Ze 339 on the growth of human PC-3 prostate adenocarcinoma cells carcinoma cells during a time period of 16 days cells in comparison to positive (Metformin) and negative (untreated and vehicle) controls.
**Fig. 5** is a chart of the results of example 2.2 demonstrating the anti-cancer effect of 3 concentrations of *Petasites* extract Ze 339 on the growth of human LS180 colorectal adenocarcinoma cells during a time period of 16 days in comparison to positive (Metformin) and negative (untreated and vehicle) controls.
**Fig. 6** is a chart of the results of example 2.3 demonstrating the anti-cancer effect of *Petasites* extract Ze 339 on the growth of human HEL erythroleukemia cells during a time period of 10 days in comparison to positive (Metformin, Ruxolitinib and Hydroxyurea) and negative (untreated and vehicle) controls.
**Fig. 7** is an isobologram generated from results of example 3.1 showing the 50% growth isobole of human HEL erythroleukemia cells 6 days after treatment with extract combinations of Ze 450 and Ze 339.
**Fig. 8** is a randomly generated isobologram to illustrate the course of antagonistic, additive and synergistic effect isoboles.

### Examples

### Materials

As exemplary extracts for illustrating the inventive effects the commercially available extracts Cimifemin^{®} (*Cimicifuga racemosa,* Ze 450, Max Zeller Soehne AG, Romanshorn, Switzerland) and Tesalin^{®} (*Petasites hybridus,* Ze 339, Max Zeller Soehne AG, Romanshorn, Switzerland) were used.

PC-3 and HEL cells were cultured in RPMI medium 1640 with GlutaMAX supplemented with FBS 10% (v/v) and Penicillin/Streptomycin. LS-180 cells were cultured in DMEM (4.5 g glucose/l) with GlutaMAX supplemented with sodium pyruvate (1 mM), MEM non-essential amino acids, FBS 10% (v/v) and Penicillin/Streptomycin. Final concentration of Penicillin/Streptomycin was 100 U/ml / 0.1 mg/ml.

HEL cells were obtained from DSMZ (Braunschweig, Germany), LS-180 and PC-3 cells were purchased from ATCC (Manassas, Virginia, USA). Culture media and supplements were purchased from Thermo-Fischer Scientific (Waltham, Massachusetts, USA). Extracts were provided by Max Zeller Soehne AG. Solvents and chemicals were purchased from Sigma-Aldrich (St. Louis, Missouri, USA) with the exception of Ruxolitinib which was provided by Selleckchem (Houston, Texas, USA).

### Example 1 - Antiproliferative effects of Cimicifuga extract (Ze 450)

### Example 1.1 - Prostate adenocarcinoma

The PC3 cell line was established in 1979 from bone metastasis of grade IV of prostate cancer in a 62-year-old Caucasian male. PC3 (PC-3) is used in prostate cancer research and drug development. PC3 cells are well-established as being useful in investigating biochemical changes in advanced prostate cancer cells and in assessing their response to chemotherapeutic agents.

A transparent flat-bottomed 96-well plate was charged with PC-3 cells (1000 cells in 95 ul growth culture Medium supplemented with Penicillin/Streptomycin per well). Cells were allowed to grow and attach overnight in humidified atmosphere at 37°C and 5 % CO₂. The next day treatment solutions were added to the wells. Cell growth was observed via measurement of confluence with a Tecan Spark multimode microplate reader. Mean confluence ± standard deviation (n = 3) was plotted against time.

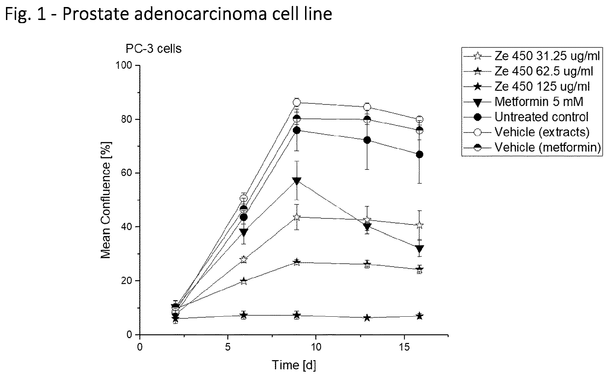

The results are summarized in Fig. 1, which demonstrates a strong concentration-dependent cell growth inhibition for the *Cimicifuga* extracts over negative control (untreated and vehicle controls) compared to the positive control (Metformin).

### Example 1.2 - Colorectal Adenocarcinoma

The LS-180 Duke's Type B colorectal adenocarcinoma cell line LS-180 is a well-established human cell line for assessing the effects of candidate compounds on adenocarcinoma cell growth.

A transparent flat-bottomed 96-well plate was charged with LS-180 cells (1000 cells in 95 ul culture medium supplemented with Penicillin/Streptomycin per well). Cells were allowed to grow and attach over night in humidified atmosphere at 37°C and 5 % CO₂. The next day indicated treatment was added to the wells. Cell growth was observed via measurement of confluence with a Tecan Spark multimode microplate reader. Mean confluence ± standard deviation (n = 3) was plotted against time.

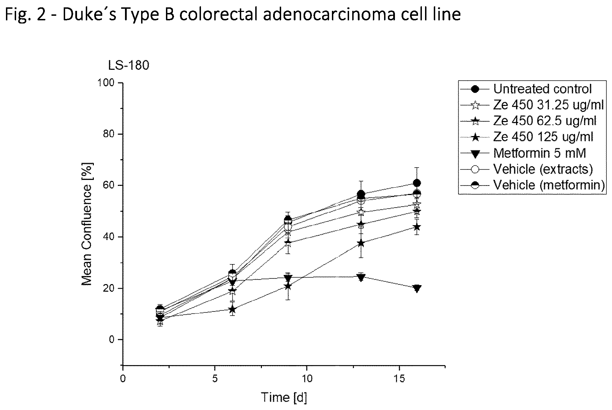

The results are summarized in Fig. 2, which demonstrates a concentration-dependent cell growth inhibition for the *Cimicifuga* extracts over negative controls (untreated and vehicle controls) compared to the positive control (Metformin).

### Example 1.3 - Erythroleukemia

The human erythroleukemia cell line HEL developed in 1982 (Paul Martin et al. Science (1982) 216(11), 1233 - 1235) is a well-established human cell line for assessing the effects of candidate compounds on erythroleukemia cell growth.

A transparent flat-bottomed 96-well plate was charged with HEL cells (1000 cells in 95 ul culture medium supplemented with Penicillin/Streptomycin per well). Cells were allowed to grow over night in humidified atmosphere at 37°C and 5 % CO₂. The next day indicated treatment was added to the wells. Cell growth was observed via measurement of optical density at 600 nm (OD600) with a Tecan Spark multimode microplate reader. Cell number was calculated from blank corrected OD600 values using a previously generated linear calibration curve from serially diluted HEL cells. Mean cell number ± standard deviation (n = 3) was plotted against time.

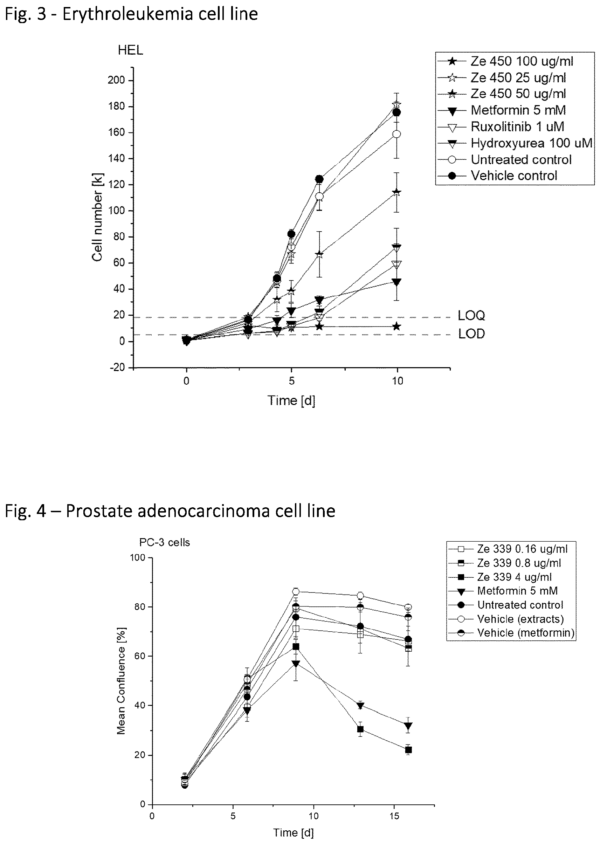

The results are summarized in Fig. 3, which demonstrates cell growth inhibition for the *Cimicifuga* extract over negative control (untreated and vehicle controls) compared to the positive controls (metformin, ruxolitinib and hydroxyurea). The *Cimicifuga* extract inhibits HEL cell growth in a concentration dependent manner and outperforms the positive controls Metformin, Ruxolitinib and Hydroxyurea. The extract at the highest tested concentration of 100 µg/ml completely prevents growth of HEL cells.

### Example 2 - Antiproliferative effects of Petasites extract (Ze 339)

### Example 2.1 - Prostate adenocarcinoma

The experimental setup was identical to that of Example 1.1.

The results are summarized in Fig. 4, which demonstrates a cell growth inhibition for the *Petasites* extract Ze 339 compared to negative (untreated and vehicle) and positive (metformin) controls. 4 µg/ml of *Petasites* extract Ze 339 shows similar effect as treatment with 5 mM metformin.

### Example 2.2 - Colorectal adenocarcinoma

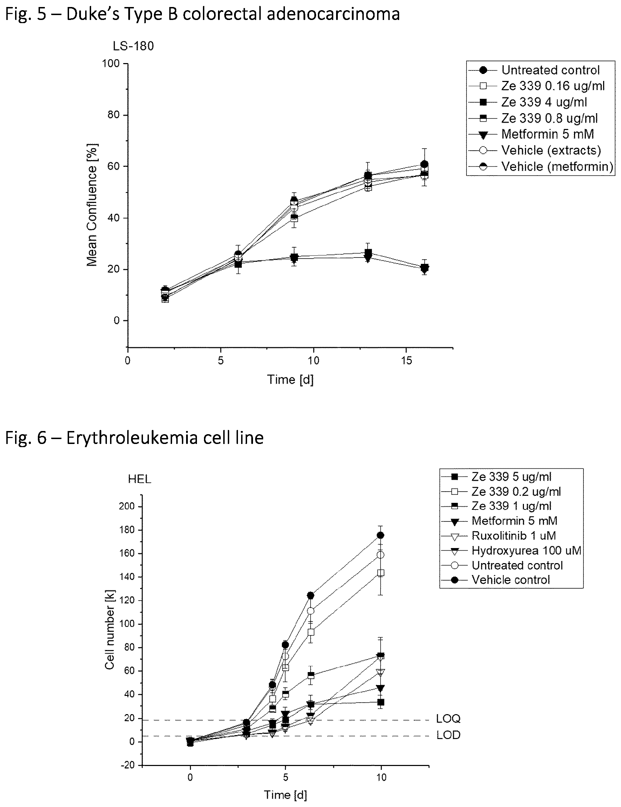

The experimental setup was identical to that of Example 1.2. The results are summarized in Fig. 5, which demonstrates a cell growth inhibition for the *Petasites* extract Ze 339 compared to negative (untreated and vehicle) and positive (metformin) control (Metformin). 4 µg/ml of *Petasites* extract Ze 339 shows similar effect as treatment with 5 mM metformin.

### Example 2.3 - Erythroleukemia

The experimental setup was identical to that of Example 1.3.

The results are summarized in Fig. 6, which demonstrates cell growth inhibition for the *Petasites* extracts over negative controls (untreated and vehicle) compared to the positive controls (metformin, ruxolitinib and hydroxyurea). The *Petasites* extracts concentration-dependently inhibit HEL cell growth. 0.2 or 1 µg/ml Ze 339 reduce growth of HEL cells to a similar extent as the positive control metformin, ruxolitinib and hydroxyurea.

### Example 3 - Antiproliferative effects of extract combinations of Cimicifuga and Petasites

Transparent flat-bottomed 96-well plates were charged with HEL cells (1000 cells in 95 ul culture medium supplemented with Penicillin/Streptomycin per well). Cells were allowed to grow over night in humidified atmosphere at 37°C and 5 % CO₂. The next day cells were treated with various concentration series of either Ze 450 or Ze 339 given alone or in combination with different fixed concentrations of either Ze 339 or Ze 450. Cell growth was observed via measurement of optical density at 600 nm (OD600) with a Tecan Spark multimode microplate reader. Cell number was calculated from blank corrected OD600 values using a previously generated linear calibration curve from serially diluted HEL cells. Extract synergism was observed after 6 days. To demonstrate synergism the growth of HEL cells after 6 days was plotted against the concentration of either Ze 450 or Ze 339 given alone or in combination with the different fixed concentrations of either Ze 339 or Ze 450 respectively. Extract combinations that resulted in 50 % growth of HEL cells were determined from curve intersects.

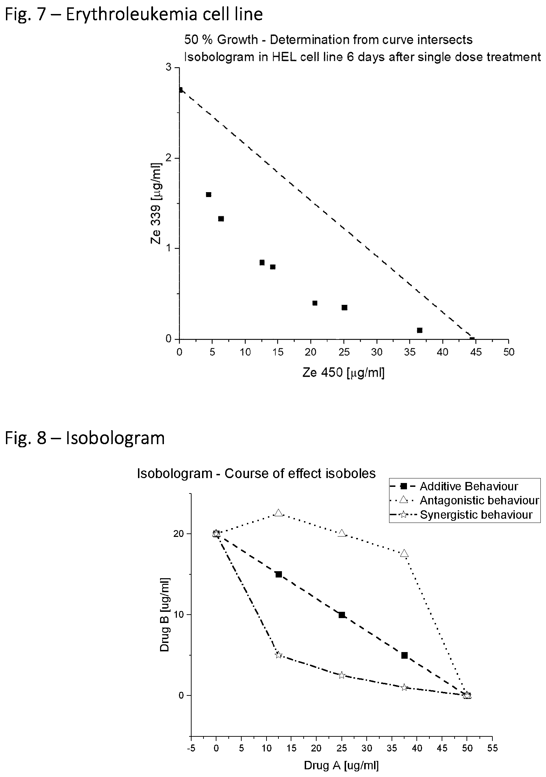

The results are summarized in Fig. 7. The 50 % growth - isobologram clearly demonstrates the synergistic antiproliferative effect of Ze 450 and Ze 339 on HEL cells as the extract combinations needed to result in 50 % growth inhibition are located clearly below the dashed line, which indicates the hypothetical location of extract combinations if the pharmacological behavior of the extract combination was additive only.

Fig. 8 explains the principle of demonstrating drug - drug synergism with isobolograms. Drug A needs to be administered at a concentration of 50 µg/ml to achieve a certain effect in a certain system. For example, one could assume Drug A to be an antibiotic and its minimal inhibitory concentration (MIC) that would prevent a certain bacterial strain from growth on an agar plate would be 50 µg/ml. Drug B would be a different antibiotic acting against the same strain of bacteria. The minimal inhibitory concentration of Drug B given alone would be 20 µg/ml. If Drug A and Drug B are combined, different outcomes on the growth of the bacterial strain could be observed in principle.

If the pharmacological effects of Drug A and Drug B would linearly add to each other, drug combinations located on the direct connection between MIC of Drug A (50 µg/ml) and MIC of Drug B (20 µg/ml) in the isobologram would result in the same pharmacological effect (squares, linear connection). The same effect on the growth of the bacteria could therefore be achieved with either 20 µg/ml Drug B alone, 50 µg/ml Drug A alone, or various combinations of Drug A and Drug B located on the linear connection between MIC of Drug A and MIC of Drug B (for example the combination of 10 µg/ml Drug B and 25 µg/ml Drug A).

If the effects of Drug A and Drug B would oppose each other, combinations of Drug A and Drug B resulting in the prevention of bacterial growth would be located *above* the direct connection of additivity between MIC of Drug A and MIC of Drug B in the isobologram (triangles). Weeks et al. (Antimicrob Agents Chemother (1981), 20, 281-285) presented a real-life example of two antagonistic drugs such as ampicillin acting bactericidal on growing bacteria combined with a bacteriostatic agent such as chloramphenicol.
On the other hand drug A and drug B could act synergistically resulting in an MIC isobole running *below* the theoretical linear line of additivity connecting MIC of Drug A and MIC of Drug B, meaning an increase in potency and/or reduction of administered dose and eventually also a hypothetical reduction of adverse drug reactions and reduced formation of resistant strains. The well-known practical example is the combination of trimethoprim and sulfamethoxazole acting on two consecutive enzymes needed for bacterial biosynthesis of tetrahydrofolic acid (see fo example Acar et al. J Infect. Dis. (1973) 128**).**

## Claims

1. An extract combination comprising extracts from *Cimicifuga sp.* and *Petasites sp.* for use in the therapeutic or prophylactic treatment of cancer.

2. The extract combination for use according to claim 1, wherein the *Cimicifuga extract* is obtained from the group of Cimicifuga plant species consisting of *C*. *racemosa L., C*. *americana* Michx., *C. foetida L (syn. A. cimicifuga), C. dahurica* (Turcz) Maxim, *C. heracleifolia* Kom., *Actea cordifolia, A. pachypoda, A. podocarpa or A. rubra.*

3. The extract combination for use according to claim 1 or 2, wherein the *Petasites* extract is obtained from the group of Petasites plant species selected from the group consisting of *Petasites hybridus, Petasites albus, Petasites amplus, Petasites fragrans Petasites formosanus, Petasites frigidus, Petasites georgicus, Petasites japonicus, Petasites laevigatus, Petasites kalbikianus, Petasites niveus, Petasites paradoxus, Petasites pyrenaicus, Petasites tricholobus, Petasites radiates, Petasites sagittatus* and *Petasites spurius,* preferably *Petasites hybridus.*

4. The extract combination for use according to any of claims 1 to 3, wherein the cancer is selected from the group consisting of malignant neoplasms of the lip, oral cavity, pharynx, digestive organs, respiratory system, intrathoracic organs, bone, articular cartilage, skin, connective tissue, soft tissue, breast, female genital organs, male genital organs, urinary organs, eye, brain and central nervous system, endocrine glands, lymphoid tissue, haematopoietic tissue, as well as benign neoplasms.

5. The extract combination for use according to any of claims 1 to 4, wherein the cancer is selected from the group consisting of polycythaemia vera (PV), myelodysplastic syndromes (MDS) and other neoplasms of lymphoid and haematopoietic tissue, such as essential thrombocythaemia (ET), primary myelofibrosis (PMF), chronic myelogenous leukemia (CML), chronic neutrophilic leukemia (CNL), juvenile myelomonocytic leukemia (JML), chronic eosinophilic leukemia (CEL), non-Hodgkin's lymphoma or multiple myeloma, as well as breast cancer, prostate cancer, pancreas cancer, hepatocellular cancer, colon cancer, lung cancer and renal cancer.

6. The extract combination for use according to any of claims 1 to 5, wherein the extract of *Cimicifuga* is prepared by a method comprising the steps of:
(i) providing plant material from *Cimicifuga sp.,* preferably *Cimicifuga racemosa,*
(ii) treating the plant material with an extraction agent, and
(iii) concentrating the extract.

7. The *Cimicifuga* extract for use according to claim 6, wherein the extract resulting from (ii) is concentrated in the presence of a solution mediator, preferably an organic polymer capable of being colloidally dispersible or soluble in the extraction agent, more preferably an organic polymer having an average molecular mass of at least about 1000 Dalton units, preferably polyvinylpyrrolidone (PVP).

8. The extract combination for use according to any of claims 1 to 7, wherein the *Petasites* extract is substantially free of pyrrolizidine-alkaloids.

9. The extract combination for use according to any of claims 1 to 7, wherein *Cimicifuga sp.* is selected from *Cimicifuga racemosa* L., *C*. *americana* Michx., *C*. *foetida* L *(syn. A. cimicifuga), C. dahurica* (Turcz) Maxim, C. *heracleifolia* Kom., *A. cordifolia, A. pachypoda, A. podocarpa and A. rubra,* preferably *C*. *racemosa,* and the extract is suitable for oral administration.

10. A pharmaceutical composition comprising a combination of an extract from *Cimicifuga sp.* and an extract from *Petasites sp.,* and optionally at least one pharmaceutically acceptable excipient for use in the therapeutic or prophylactic treatment of cancer.

11. The pharmaceutical composition for use according to claim 10, wherein the *Cimicifuga* extract is obtained from the group of Cimicifuga plant species consisting of *C*. *racemosa* L*., C. americana* Michx., *C*. *foetida* L *(syn. A. Cimicifuga), C. dahurica* (Turcz) Maxim, *C*. *Heracleifolia* Kom., *A. cordifolia, A. pachypoda, A. podocarpa* and *A. Rubra* and/or the *Petasites extract* is obtained from the group of Petasites plant species selected from the group consisting of *Petasites hybridus, Petasites albus, Petasites amplus, Petasites fragrans Petasites formosanus, Petasites frigidus, Petasites georgicus, Petasites japonicus, Petasites laevigatus, Petasites kalbikianus, Petasites niveus, Petasites paradoxus, Petasites pyrenaicus, Petasites tricholobus, Petasites radiates, Petasites sagittatus* and *Petasites spurius.*

12. The pharmaceutical composition for use according to claim 10 or 11, wherein the cancer is selected from the group consisting of polycythaemia vera (PV), myelodysplastic syndromes (MDS) and other neoplasms of lymphoid and haematopoietic tissue, such as essential thrombocythaemia (ET), primary myelofibrosis (PMF), chronic myelogenous leukemia (CML), chronic neutrophilic leukemia (CNL), juvenile myelomonocytic leukemia (JML), chronic eosinophilic leukemia (CEL), non-Hodgkin's lymphoma or multiple myeloma, as well as breast cancer, prostate cancer, pancreas cancer, hepatocellular cancer, colon cancer, lung cancer and renal cancer.

13. The pharmaceutical composition for use according to any of claims 10 to 12, wherein the extract combination is for oral administration.

## Patentansprüche

1. Eine Extraktkombination, die Extrakte aus *Cimicifuga sp.* und *Petasites sp.* umfasst, zur Verwendung in der therapeutischen oder prophylaktischen Behandlung von Krebs.

2. Die Extraktkombination zur Verwendung entsprechend Anspruch 1, wobei der *Cimicifuga-*Extrakt aus der Gruppe der Cimicifuga-Pflanzenarten gewonnen wird, die aus C. *racemosa* L*.*, *C*. *americana* Michx., *C. foetida* L *(syn. A. cimicifuga), C. dahurica* (Turcz) Maxim, *C. heracleifolia* Kom., *Actea cordifolia, A. pachypoda, A. podocarpa oder A. rubra* besteht.

3. Die Extraktkombination zur Verwendung entsprechend Anspruch 1 oder 2, wobei der *Peta*sites-Extrakt aus der Gruppe der Petasites-Pflanzenarten gewonnen wird, die aus der Gruppe ausgewählt sind, bestehend aus *Petasites hybridus, Petasites albus, Petasites amplus, Petasites fragrans Petasites formosanus, Petasites frigidus, Petasites georgicus, Petasites japonicus, Petasites laevigatus, Petasites kalbikianus, Petasites niveus, Petasites paradoxus, Petasites pyrenaicus, Petasites tricholobus, Petasites radiates, Petasites sagittatus* and *Petasites spurius,* preferably *Petasites hybridus.*

4. Die Extraktkombination zur Verwendung entsprechend einem der Ansprüche 1 bis 3, wobei der Krebs aus der Gruppe ausgewählt ist, die aus malignen Neoplasmens der Lippe, der Mundhöhle, des Rachens, der Verdauungsorgane, des Atmungssystems, der intrathorakalen Organe, der Knochen, des Gelenkknorpels, der Haut, des Bindegewebes, des Weichgewebes, der Brust, der weiblichen Geschlechtsorgane, der männlichen Geschlechtsorgane, der Harnorgane, des Auges, des Gehirns und des zentralen Nervensystems, der endokrinen Drüsen, des Lymphgewebes, des hämatopoetisches Gewebes sowie gutartigen Neoplasmen besteht.

5. Die Extraktkombination zur Verwendung entsprechend einem der Ansprüche 1 bis 4, wobei der Krebs aus der Gruppe ausgewählt ist, die aus Polyzythämie vera (PV), myelodysplastischen Syndromen (MDS) und anderen Neoplasien des lymphatischen und hämatopoetischen Gewebes, wie essenzieller Thrombozythämie (ET), primärer Myelofibrose (PMF), chronischer myeloischer Leukämie (CML), chronischer neutrophiler Leukämie (CNL), juveniler myelomonozytischer Leukämie (JML), chronischer eosinophiler Leukämie (CEL), Non-Hodgkin-Lymphom oder multiplem Myelom, sowie Brustkrebs, Prostatakrebs, Bauchspeicheldrüsenkrebs, Leberkrebs, Darmkrebs, Lungenkrebs und Nierenkrebs besteht.

6. Die Extraktkombination zur Verwendung entsprechend einem der Ansprüche 1 bis 5, wobei der Extrakt aus *Cimicifuga* durch ein Verfahren hergestellt wird, das die folgenden Schritte umfasst:
(i) Bereitstellen von Pflanzenmaterial aus *Cimicifuga sp.,* vorzugsweise *Cimicifuga racemosa,*
(ii) Behandeln des Pflanzenmaterials mit einem Extraktionsmittel und
(iii) Konzentrieren des Extrakts.

7. Der *Cimicifuga-*Extrakt zur Verwendung entsprechend Anspruch 6, wobei der aus (ii) resultierende Extrakt in der Gegenwart eines Lösungsvermittlers konzentriert wird, vorzugsweise eines organischen Polymers, das in dem Extraktionsmittel kolloidal dispergierbar oder löslich ist, mehr bevorzugt eines organischen Polymers mit einer durchschnittlichen Molekülmasse von mindestens etwa 1000 Dalton-Einheiten, vorzugsweise Polyvinylpyrrolidon (PVP).

8. Die Extraktkombination zur Verwendung entsprechend einem der Ansprüche 1 bis 7, wobei der Petasites-Extrakt im Wesentlichen frei von Pyrrolizidinalkaloiden ist.

9. Die Extraktkombination zur Verwendung entsprechend einem der Ansprüche 1 bis 7, wobei *Cimicifuga sp.* aus *Cimicifuga racemosa* L., *C*. *americana* Michx., *C*. *foetida* L *(syn. A. cimicifuga), C. dahurica* (Turcz) Maxim, C. *heracleifolia* Kom., *A. cordifolia, A. pachypoda, A. podocarpa and A. rubra,* vorzugsweise C. *racemosa,* ausgewählt ist, und der Extract zur oralen Verabreichung geeignet ist.

10. Eine pharmazeutische Zusammensetzung, umfassend eine Kombination aus einem Extrakt aus *Cimicifuga sp.* und einem Extrakt aus *Petasites sp.* und optional wenigstens einen pharmazeutisch verträglichen Hilfsstoff zur Verwendung in der therapeutischen oder prophylaktischen Behandlung von Krebs.

11. Die pharmazeutische Zusammensetzung zur Verwendung entsprechend Anspruch 10, wobei der *Cimicifuga-*Extrakt aus der Gruppe der Cimicifuga-Pflanzenarten gewonnen wird, die aus *C*. *racemosa* L*., C. americana* Michx., *C. foetida* L *(syn. A. Cimicifuga), C. dahurica* (Turcz) Maxim, *C*. *Heracleifolia* Kom., *A. cordifolia, A. pachypoda, A. podocarpa* und *A. Rubra* besteht und/oder der *Petasites-Extrakt* aus der Gruppe der Petasites-Pflanzenarten gewonnen wird, die aus *Petasites hybridus, Petasites albus, Petasites amplus, Petasites fragrans Petasites formosanus, Petasites frigidus, Petasites georgicus, Petasites japonicus, Petasites laevigatus, Petasites kalbikianus, Petasites niveus, Petasites paradoxus, Petasites pyrenaicus, Petasites tricholobus, Petasites radiates, Petasites sagittatus* and *Petasites spurius* besteht.

12. Die pharmazeutische Zusammensetzung zur Verwendung entsprechend Anspruch 10 oder 11, wobei der Krebs aus der Gruppe ausgewählt ist, die aus Polyzythämie vera (PV), myelodysplastischen Syndromen (MDS) und anderen Neoplasien des lymphatischen und hämatopoetischen Gewebes, wie essenzieller Thrombozythämie (ET), primärer Myelofibrose (PMF), chronischer myeloischer Leukämie (CML), chronischer neutrophiler Leukämie (CNL), juveniler myelomonozytischer Leukämie (JML), chronischer eosinophiler Leukämie (CEL), Non-Hodgkin-Lymphom oder multiplem Myelom, sowie Brustkrebs, Prostatakrebs, Bauchspeicheldrüsenkrebs, Leberkrebs, Darmkrebs, Lungenkrebs und Nierenkrebs besteht.

13. Die pharmazeutische Zusammensetzung zur Verwendung entsprechend einem der Ansprüche 10 bis 12, wobei die Extraktkombination zur oralen Verabreichung vorgesehen ist.

## Revendications

1. Combinaison d'extraits comprenant des extraits de *Cimicifuga sp.* et de *Petasites sp*. pour une utilisation dans le traitement thérapeutique ou prophylactique du cancer.

2. Combinaison d'extraits pour une utilisation selon la revendication 1, dans laquelle l'extrait de *Cimicifuga* est obtenu à partir du groupe d'espèces végétales Cimicifuga constitué de *C. racemosa* L*., C*. *americana* Michx., *C. foetida* L *(syn. A. cimicifuga), C. dahurica* (Turcz) Maxim, *C*. *heracleifolia* Kom., *Actea cordifolia, A. pachypoda, A. podocarpa ou A. rubra.*

3. Combinaison d'extraits pour une utilisation selon la revendication 1 ou 2, dans laquelle l'extrait de *Petasites* est obtenu à partir du groupe d'espèces végétales Petasites choisies parmi le groupe constitué de *Petasites hybridus, Petasites albus, Petasites amplus, Petasites fragrans, Petasites formosanus, Petasites frigidus, Petasites georgicus, Petasites japonicus, Petasites laevigatus, Petasites kalbikianus, Petasites niveus, Petasites paradoxus, Petasites pyrenaicus, Petasites tricholobus, Petasites radiates, Petasites sagittatus* et *Petasites spurius,* de préférence *Petasites hybridus.*

4. Combinaison d'extraits pour une utilisation selon l'une quelconque des revendications 1 à 3, dans laquelle le cancer est choisi parmi le groupe constitué de néoplasmes malins de la lèvre, de la cavité buccale, du pharynx, des organes digestifs, du système respiratoire, des organes intrathoraciques, des os, du cartilage articulaire, de la peau, du tissu conjonctif, des tissus mous, du sein, des organes génitaux féminins, des organes génitaux masculins, des organes urinaires, de l'œil, du cerveau et du système nerveux central, des glandes endocrines, du tissu lymphoïde, du tissu hématopoïétique, ainsi que de néoplasmes bénins.

5. Combinaison d'extraits pour une utilisation selon l'une quelconque des revendications 1 à 4, dans laquelle le cancer est choisi parmi le groupe constitué de la polycythémie vraie (PV), des syndromes myélodysplasiques (SMD) et d'autres néoplasmes des tissus lymphoïdes et hématopoïétiques, tels que la thrombocythémie essentielle (TE), la myélofibrose primitive (MP), la leucémie myéloïde chronique (LMC), la leucémie neutrophile chronique (LNC), la leucémie myélomonocytaire juvénile (LMJ), la leucémie éosinophile chronique (LEC), le lymphome non hodgkinien ou le myélome multiple, ainsi que le cancer du sein, le cancer de la prostate, le cancer du pancréas, le cancer hépatocellulaire, le cancer du côlon, le cancer du poumon et le cancer du rein.

6. Combinaison d'extraits pour une utilisation selon l'une quelconque des revendications 1 à 5, dans laquelle l'extrait de *Cimicifuga* est préparé par un procédé comprenant les étapes de :
(i) fourniture du matériel végétal issu de *Cimicifuga sp.,* de préférence *Cimicifuga racemosa,*
(ii) traitement du matériel végétal avec un agent d'extraction, et
(iii) concentration de l'extrait.

7. Extrait de *Cimicifuga* pour une utilisation selon la revendication 6, dans lequel l'extrait résultant de (ii) est concentré en présence d'un médiateur de solution, de préférence un polymère organique capable d'être dispersible ou soluble colloïdalement dans l'agent d'extraction, plus préférentiellement un polymère organique présentant une masse moléculaire moyenne d'au moins environ 1000 unités Dalton, de préférence la polyvinylpyrrolidone (PVP).

8. Combinaison d'extraits pour une utilisation selon l'une quelconque des revendications 1 à 7, dans laquelle l'extrait de *Petasites* est sensiblement exempt d'alcaloïdes pyrrolizidiniques.

9. Combinaison d'extraits pour une utilisation selon l'une quelconque des revendications 1 à 7, dans laquelle *Cimicifuga sp.* est sélectionné parmi *Cimicifuga racemosa* L*., C. americana* Michx., *C. foetida L (syn. A. cimicifuga), C. dahurica* (Turcz) Maxim, C. *heracleifolia* Kom., *A. cordifolia, A. pachypoda, A. podocarpa et A. rubra,* de préférence C. *racemosa,* et l'extrait convient à une administration orale.

10. Composition pharmaceutique comprenant une combinaison d'un extrait de *Cimicifuga sp.* et d'un extrait de *Petasites sp.,* et éventuellement au moins un excipient pharmaceutiquement acceptable pour une utilisation dans le traitement thérapeutique ou prophylactique du cancer.

11. Composition pharmaceutique pour une utilisation selon la revendication 10, dans laquelle l'extrait de *Cimicifuga* est obtenu à partir du groupe d'espèces végétales Cimicifuga constitué de *C*. *racemosa* L*., C. americana* Michx., *C. foetida* L *(syn. A. Cimicifuga), C. dahurica* (Turcz) Maxim, *C*. *Heradeifolia* Kom., *A. cordifolia, A. pachypoda, A. podocarpa* et *A. rubra* et/ou l'extrait de *Petasites* est obtenu à partir du groupe d'espèces végétales Petasites choisies parmi le groupe constitué de *Petasites hybridus, Petasites albus, Petasites amplus, Petasites fragrans, Petasites formosanus, Petasites frigidus, Petasites georgicus, Petasites japonicus, Petasites laevigatus, Petasites kalbikianus, Petasites niveus, Petasites paradoxus, Petasites pyrenaicus, Petasites tricholobus, Petasites radiates, Petasites sagittatus* et *Petasites spurius.*

12. Composition pharmaceutique pour une utilisation selon la revendication 10 ou 11, dans laquelle le cancer est choisi parmi le groupe constitué de la polycythémie vraie (PV), des syndromes myélodysplasiques (SMD) et d'autres néoplasmes des tissus lymphoïdes et hématopoïétiques, tels que la thrombocythémie essentielle (TE), la myélofibrose primitive (MP), la leucémie myéloïde chronique (LMC), la leucémie neutrophile chronique (LNC), la leucémie myélomonocytaire juvénile (LMJ), la leucémie éosinophile chronique (LEC), le lymphome non hodgkinien ou le myélome multiple, ainsi que le cancer du sein, le cancer de la prostate, le cancer du pancréas, le cancer hépatocellulaire, le cancer du côlon, le cancer du poumon et le cancer du rein.

13. Composition pharmaceutique pour une utilisation selon l'une quelconque des revendications 10 à 12, dans laquelle la combinaison d'extraits convient à une administration orale.
